# EUROPÄISCHE PATENTSCHRIFT

(11) **EP 0 982 303 B1**
(45) Veröffentlichungstag und Bekanntmachung des Hinweises auf die Patenterteilung: **13.02.2002**
(21) Anmeldenummer: 99107946.8
(22) Anmeldetag: 22.04.1999
(51) Int. Cl.: C07D 319/06, A61K 7/46, C11D 3/39

(54) **2,4,6-Trimethyl-4-phenyl -1,3-dioxan**
2,4,6-Trimethyl-4-phenyl-1,3-dioxane
2,4,6-Trimethyl-4-phenyl-1,3-dioxan

(30) Priorität: 07.05.1998 DE 19822232
(43) Veröffentlichungstag der Anmeldung: 01.03.2000
(73) Patentinhaber: Dragoco Gerberding & Co Aktiengesellschaft, 37603 Holzminden (DE)
(72) Erfinder: Kappey, Claus-Hermann, 37603 Holzminden (DE); Hölscher, Bernd, 37620 Halle (DE); Pickenhagen, Wilhelm Dr., 37671 Höxter (DE)
(74) Vertreter: Eisenführ, Speiser & Partner

(56) Entgegenhaltungen:
- US-A- 4 198 323
- CHEMICAL ABSTRACTS, vol. 95, no. 11, 14. September 1981 (1981-09-14) Columbus, Ohio, US; abstract no. 97691y, EL GHARBI, R. ET AL.: "Condensation of substituted styrenes with aliphatic and aromatic aldehydes; an extension of the Prins reaction" Seite 646; Spalte 1; XP002900727 & SYNTHESIS 1981, (5), PAGES 361-362 ,
- CHEMICAL ABSTRACTS, vol. 100, no. 9, 27. Februar 1984 (1984-02-27) Columbus, Ohio, US; abstract no. 68236v, EL GHARBI, R. ET AL.: "Condensation of aromatic alkenes with acetaldehyde catalyzed by ion-exchange resins. II. Stereoisomerization and synthesis of new polysubstituted 1,3-dioxacyclohexanes" Seite 594; Spalte 2; XP002900728 & TETRAHEDRON, Bd. 39, Nr. 18, 1983, Seiten 2953-2963,
- CHEMICAL ABSTRACTS, vol. 108, no. 10, 7. März 1988 (1988-03-07) Columbus, Ohio, US; abstract no. 77681d, IZUMI, YU ET AL.: "Liquid hypochlorite bleaching compositions containing odor-masking cyclic ethers" Seite 77690; Spalte 1; XP002900729 & JP 62 205200 A (JPN. KOKAI TOKKYO KOHO)
- CHEMICAL ABSTRACTS, vol. 64, no. 8, 11. April 1966 (1966-04-11) Columbus, Ohio, US; abstract no. 11217f, SANYO CHEMICAL INDUSTRY CO., LTD.: "1,3-Dioxanes" Seite 11217; Spalte 1; XP002900730 & JP 66 100102 A (SANYO CHEMICAL CO., LTD.)
- CHEMICAL ABSTRACTS, vol. 102, no. 26, 1. Juli 1985 (1985-07-01) Columbus, Ohio, US; abstract no. 225585u, OGAWA AND CO., LTD.: "Fragrant deodorizing mixtures" Seite 312; Spalte 2; XP002900731 & JP 60 014859 A (OGAWA AND CO., LTD.)

## Beschreibung

Die vorliegende Erfindung betrifft 2,4,6-Trimethyl-4-phenyl-1,3-dioxan der allgemeinen Formel C.

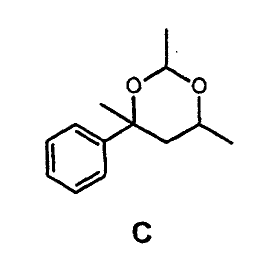

In der Riechstoffindustrie besteht ein stetiger Bedarf an neuen Riechstoffen. Die Verwendung von 2,4,6-Trimethyl-4-phenyl-1,3-dioxan der allgemeinen Formel C in Riechstoffmischungen ist bereits bekannt und wird in den Patentdokumenten WO 9629281 (The Procter & Gamble Company, USA), JP 62205200 (Kao Corp., Japan) und JP 60014859 (Ogawa and Co., Ltd., Japan) referiert. Angaben über isomere Formen des 1,3-Dioxans C bzw. deren spezifische sensorische Eigenschaften enthalten die vorgenannten Schriften nicht.

In den Chemical Abstracts (Chem. Abstr. Vol. 64, 11217f (1966)) wird ein Patent der Sanyo Chemical Industry Co. (von K. Uno et al., Japan. 102('66), Jan 7, Appl. Nov. 28, 1962) referiert, das die Darstellung von 1,3-Dioxanen aus aliphatischen Aldehyden mit Olefinen unter Tonerde-Katalyse zum Gegenstand hat. Als Beispiel wird die Umsetzung von 2 Moläquivalenten Paraldeyd (entsprechend 6 Moläquivalenten Acetaldehyd) mit 3 Moläquivalenten Isobuten in Gegenwart von saurer Tonerde zu 2,4,4,6-Tetramethyl-1,3-dioxan angeführt. Als auf ähnliche Weise zugänglich wird auch das 2,4,6-Trimethyl-4-phenyl-1,3-dioxan der allgemeinen Formel C genannt, was für den Fachmann bedeutet, daß das 1,3-Dioxan der allgemeinen Formel C aus den entsprechenden Mengen von α-Methylstyrol (A) und Paraldehyd (B) in Gegenwart von saurer Tonerde synthetisiert wurde.

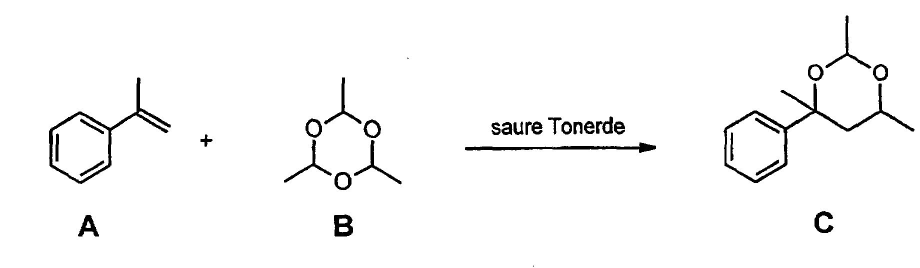

R. el Gharbi et al. (Synthesis 1971, 361-2; Tetrahedron Vol. 39, 2953-63 (1983)) beschreiben Gemische von zwei isomeren Formen (diastereomere Enantiomerenpaare 1 und 2) des 2,4,6-Trimethyl-4-phenyl-1,3-dioxans. Die Isomere (2RS,4SR,6RS)-2,4,6-Trimethyl-4-phenyl-1,3-dioxan (1) und (2RS,4RS,6RS)-2,4,6-Trimethyl-4-phenyl-1,3-dioxan (2) werden in leicht unterschiedlichem Verhältnis durch Kondensation von α-Methylstyrol (A) mit zwei Moläquivalenten Acetaldehyd unter der Katalyse von stark sauren Kationenaustauscher-Harzen (Lewatit® SP 120, Bayer AG) in Abhängigkeit von Lösemittel und Reaktionszeit und -temperatur gebildet. Für die Umsetzung in Toluol bei 20°C wird nach 24 h ein Verhältnis 1/2 von 68:32 genannt; für die Umsetzung in Hexan bei 20°C nach 1 h bzw. bei 50°C nach 0,5 h wird das Verhältnis 1/2 mit 62:38 bzw. 65:35 angegeben. Der Fachmann leitet hieraus zwanglos ab, daß unter den vorbeschriebenen Reaktionsbedingungen das thermodynamische Gleichgewicht z.B. bei der Umsetzung in Toluol nach 24 h bei einem 68:32-Verhältnis der Substanzen 1 und 2 erreicht wurde.

R. el Gharbi et al merken an, daß die Kondensation eines Alkens mit aliphatischen und aromatischen Aldehyden - mit Ausnahme von Formaldehyd - in Gegenwart von Schwefelsäure generell zu einer komplexen Produktmischung (Glykole, Dimere, Crotonisierung des Aldehyds, Polymere etc.) führt, so daß damit dieser Katalysator als ungeeignet zur Erzielung guter Ausbeuten an den entsprechend substituierten 1,3-Dioxanen erscheint.

Angaben zur Sensorik von 1 und 2 bzw. deren Mischungen sind in den beiden vorgenannten Veröffentlichungen nicht enthalten.

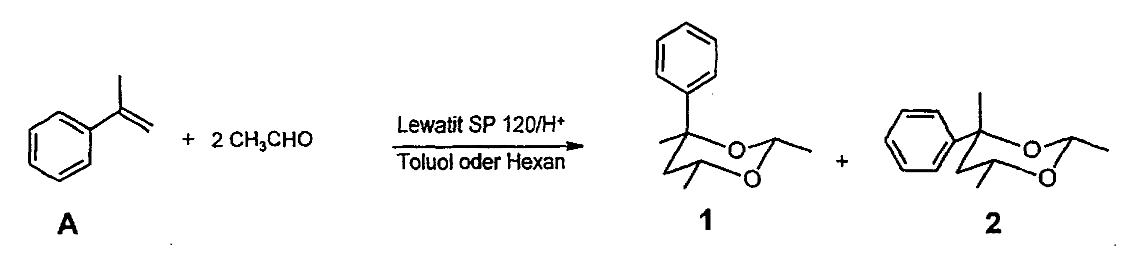

Ein Gemisch isomerer 2,4,6-Trimethyl-4-phenyl-1,3-dioxane, das als Hauptkomponenten die Isomeren 1 und 2 enthält, wird seit mehr als zwanzig Jahren als Riechstoff verwendet und unter den Namen *Vertacetal* (Hersteller: Dragoco Gerberding & Co. AG, Holzminden) und *Floropal/Corps 717* (Hersteller: Haarmann & Reimer GmbH, Holzminden) gehandelt.

Die beiden Handelsprodukte unterscheiden sich - wie jetzt eine interne Analyse ergab - bezüglich ihres Gehaltes an den Hauptkomponenten 1 und 2, aber es sind auch hinsichtlich des Gehaltes an den jetzt identifizierten, jeweils vorhandenen Nebenkomponenten (2RS,4SR,6SR)-2,4,6-Trimethyl-4-phenyl-1,3-dioxan (3) und (2SR,4SR,6RS)-2,4,6-Trimethyl-4-phenyl-1,3-dioxan (4) sowie an den Nebenprodukten 3,6-Dihydro-cis-2,6-dimethyl-4-phenyl-2H-pyran (5) und 3,6-Dihydro-trans-2,6-dimethyl-4-phenyl-2H-pyran (6) Unterschiede feststellbar (vergleiche dazu die nachfolgende "Vergleichsanalyse"). Die Verbindungen 3, 4 und 6 waren bisher nicht bekannt, sie werden weiter unten im Rahmen von Beispielen näher beschrieben und spektroskopisch charakterisiert.

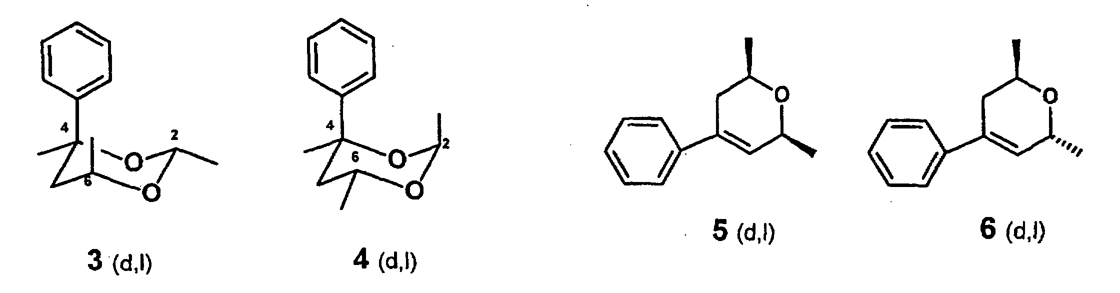

In den zugehörigen Produktdatenblättern wird der Geruch von Vertacetal mit "frisch-krautig, typischer Eindruck von Grapefruit" beschrieben, und der Geruch von *Floropal/Corps 717* wird als "krautig-frisch, blumig-grün ähnlich Chrysantheme, Cyprus und Grapefruit" angegeben. Eine sensorische Einzelbeschreibung der beiden Hauptkomponenten 1 und 2 sowie der Nebenkomponenten 3, 4, 5 und 6 lag bislang nicht vor.

Der vorliegenden Erfindung lag die Aufgabe zugrunde, einen Riechstoff anzugeben, der hinsichtlich seiner Geruchs-Charakteristik den bekannten Gemischen isomerer 2,4,6-Trimethyl-4-phenyl-1,3-dioxane zumindest in etwa entsprechen, aber möglichst noch intensiver als diese riechen sollte.

Diese Aufgabe wird durch die Angabe der Verbindungen
(2RS,4SR,6RS)-2,4,6-Trimethyl-4-phenyl-1,3-dioxan (1) und
(2RS,4SR,6SR)-2,4,6-Trimethyl-4-phenyl-1,3-dioxan (3)
als Riechstoff gelöst.

Die Erfindung beruht zunächst auf der überraschenden Erkenntnis, daß in den bekannten Gemischen isomerer 2,4,6-Trimethyl-4-phenyl-1,3-dioxane das (2RS,4SR,6RS)-2,4,6-Trimethyl-4-phenyl-1,3-dioxan (1) als aktive Riechstoffkomponente wirkt, während die weitere Hauptkomponente 2 im Gemisch mit der Komponente 1 sogar einen nachteiligen Einfluß auf die Sensorik haben kann und sich daher bei einem Einsatz in der Parfümerie nachteilig auswirken kann (vergleiche dazu nachfolgend Beispiel 5). Aus der Reihe der Nebenkomponenten 3, 4, 5 und 6 verfügt zudem überraschenderweise auch die Verbindung 3, das (2RS,4SR,6SR)-2,4,6-Trimethyl-4-phenyl-1,3-dioxan, über Riechstoffeigenschaften; ihre Geruchscharakteristik entspricht weitgehend der von 1.

Als Riechstoff besonders gut einsetzbar ist das reine (2RS,4SR,6RS)-2,4,6-Trimethyl-4-phenyl-1,3-dioxan (1), das beispielsweise durch eine Destillation aus einem Gemisch der Substanzen 1 und 2 abgetrennt werden kann. Im Vergleich zu den bisher bekannten (handelsüblichen) Riechstoffgemischen ist aber auch schon die Verwendung neuer Riechstoffgemische vorteilhaft, die einen erhöhten Anteil an (2RS,4SR, 6RS)-2,4,6-Trimethyl-4-phenyl-1,3-dioxan (1) besitzen. Derartige Riechstoffgemische mit einem Anteil an (2RS,4SR,6RS)-2,4,6-Trimethyl-4-phenyl-1,3-dioxan (1) von vorteilhafterweise über 80 Gewichtsprozent oder einem bevorzugten Anteilsverhältnis der Substanzen 1 und 2 von mindestens 4:1 lassen sich durch eine beispielsweise destillative Weiterbehandlung der auf üblichem Wege hergestellten, handelsüblichen Riechstoffgemische erhalten.

Die Vorteile bei der Verwendung der reinen Substanz 1 oder von Gemischen mit einem erhöhten Anteil an 1 werden besonders deutlich durch einen Vergleich der per Triangeltest ermittelten Geruchsschwellenwerte der Verbindungen 1 und 2.

| Geruchsschwellenwerte: | |
|---|---|
| Verbindung 1 | 0,85 µg/l Wasser |
| Verbindung 2 | 332 µg/l Wasser |

Die Verwendung von (2RS,4SR,6RS)-2,4,6-Trimethyl-4-phenyl-1,3-dioxan (1) oder hochkonzentrierten Gemischen mit der Substanz 1 als Hauptkomponente trägt durch den Fortfall bzw. die Reduzierung des Anteils sensorisch inaktiver oder nachteiliger Ballast-Komponenten zudem zu einer Verringerung der Transport- und Lagerkosten bei.

Im Rahmen der Erfindung haben sich auch hinsichtlich der Herstellungsverfahren eine Reihe neuer Aspekte ergeben.

So wurde überraschenderweise gefunden, daß sich in guter Ausbeute ein Gemisch der Substanzen 1 und 2 (neben geringen Mengen an Nebenkomponenten) mit einem vorteilhaften Anteilsverhältnis 1/2 ≥ 80 : 20 direkt aus α-Methylstyrol und Paraldehyd oder Acetaldehyd unter katalytischer Einwirkung von Brönstedt-Säuren, vorzugsweise von wässeriger Schwefelsäure, synthetisieren läßt, ohne daß es zu der befürchteten, generellen Ausbildung einer komplexen Produktmischung durch Einwirkung von Schwefelsäure kommt. Als alternative Katalysatoren können auch vorzugsweise verdünnte andere starke Brönstedt-Säuren verwendet werden, wie zum Beispiel Perchlorsäure, Salzsäure, Phosphorsäure oder p-Toluolsulfonsäure. In einem erfindungsgemäßen Verfahren zur Herstellung eines Gemisches der Verbindungen (2RS,4SR,6RS)-2,4,6-Trimethyl-4-phenyl-1,3-dioxan (1) und (2RS,4RS,6RS)-2,4,6-Trimethyl-4-phenyl-1,3-dioxan (2) werden daher α-Methylstyrol und Paraldehyd oder Acetaldehyd unter Einwirkung von Brönstedt-Säuren, vorzugsweise von wässeriger Schwefelsäure bei 20 - 30°C, umgesetzt (vgl. das Beispiel 3 weiter unten).

Gemäß einem weiteren erfindungsgemäßen Verfahren zur Herstellung von (2RS,4SR,6RS)-2,4,6-Trimethyl-4-phenyl-1,3-dioxan (1) wird ein (beispielsweise handelsübliches) Gemisch der Verbindungen 1 und 2 (mit Nebenprodukten) mit Säure behandelt, vorzugsweise mit einer Brönstedt-Säure bei 20 - 30°C. Die Verbindung 2 (und gleichermaßen die Nebenverbindungen 3 und 4) wird dabei zumindest teilweise in die Verbindung 1 umgelagert. Als Katalysator ist Schwefelsäure bevorzugt, es können aber auch andere starke Brönstedt-Säuren eingesetzt werden, wie zum Beispiel Perchlorsäure, Salzsäure, Phosphorsäure oder p-Toluolsulfonsäure. Auch Lewis-Säuren wie zum Beispiel Bortrifluorid, Aluminiumchlorid, Zinkchlorid oder Titan(IV)-chlorid können als Katalysator eingesetzt werden. Dieses erfindungsgemäße Verfahren führt überraschend ebenfalls zu Gemischen mit einem hohen Anteil an der Substanz 1; das Verhältnis der Substanzen 1 und 2 beträgt in einem typischen Beispielsfall 84:16, Verhältnisse von mindestens 80:20 lassen sich regelmäßig erreichen (vgl. unten Beispiel 4, Alternative a).

In einem alternativen erfindungsgemäßen Verfahren zur Herstellung von (2RS,4SR,6RS)-2,4,6-Trimethyl-4-phenyl-1,3-dioxan (1) wird (2RS,4RS,6RS)-2,4,6-Trimethyl-4-phenyl-1,3-dioxan (2) als Ausgangsmaterial eingesetzt und vorzugsweise mit einer Säure behandelt (vgl. unten Beispiel 4, Alternative b). Es findet hierbei ebenfalls eine Umlagerung statt, die zu einem Gemisch mit einem hohen Anteil an der Substanz 1 führt. Das Ausgangsmaterial (2RS,4RS,6RS)-2,4,6-Trimethyl-4-phenyl-1,3-dioxan (2) kann beispielsweise durch eine Destillation aus einem Gemisch der Substanzen 1 und 2 abgetrennt werden.

Nachfolgend wird die Erfindung anhand einer Vergleichsanalyse handelsüblicher Produkte und anhand erfindungsgemäßer Beispiele näher erläutert:

### Vergleichsanalyse:

### Analyse handelsüblicher Gemische mit einem Anteil an (2RS,4SR,6RS)-2,4,6-Trimethyl-4-phenyl-1,3-dioxan (1):

Zur Charakterisierung handelsüblicher Produkte wurden zwei Muster-Gemische unterschiedlicher Herkunft (siehe Tabelle) gaschromatographisch unter Verwendung einer 60 m-Glaskapillarsäule (DBWax, 50 - 200°C, 4°C/min) analysiert:
t_{R} = 30.0 (1), 31.75 (3), 31.8 (4), 33.75 (2), 35.2 (5), 36.7 min (6).

Die Zuordnung erfolgte per GC/MS-Analyse durch Korrelation mit den reinen Komponenten bzw. einem 11:1-Gemisch von 3/4.

**Tabelle 1**

| Gefundene Verteilung (Flächen-%) der Verbindungen **1** - **6**: | | | | | | |
|---|---|---|---|---|---|---|
| Handelsmuster/Verbindung | **1** | **2** | **3** | **4** | **5** | **6** |
| *Vertacetal* (Dragoco Gerberding & Co. AG) | 54,38 | 44,33 | 1,06 | 0,10 | 0,13 | - |
| *Floropal/Corps 717* (Haarmann & Reimer GmbH) | 63,71 | 34,52 | 0,76 | 0,17 | 0,80 | 0,04 |

### Beispiel 1:

### Destillative Abtrennung von (2RS,4SR,6RS)-2,4,6-Trimethyl-4-phenyl-1,3-dioxan (1):

3500 g Vertacetal (handelsübliches Produkt der Dragoco Gerberding & Co. AG, GC-Analyse siehe die vorstehende Vergleichsanalyse, Tabelle 1) wurden an einer 1,5 m-Metallfüllkörperkolonne (BX-Packungen, Fa. Sulzer) bei einem Druck von 9 - 9,5 hPa fraktionierend destilliert.
(a) Im Siedebereich 99 - 101°C fielen 1868 g (2RS,4SR,6RS)-2,4,6-Trimethyl-4-phenyl-1,3-dioxan (1) als farblose Flüssigkeit an; GC-Reinheit: 99,9 %.
   Es ergab sich das folgende Analyseergebnis für (2RS,4SR,6RS)-2,4,6-Trimethyl-4-phenyl-1,3-dioxan (1) :
   Geruch: stark, krautig-frisch, grün, typisch Grapefruit.
   ¹H-NMR (300 MHz, CDCl₃): δ = 1.21 (d, J = 6.5 Hz, 3 H), 1.33 (d, J = 5 Hz, 3 H), 1.40 (s, 3 H), 1.67 (dd, J = 11.5 Hz, J = 14 Hz, 1 H), 2.32 (dd, J = 2 Hz, J = 14 Hz, 1 H), 3.65 (ddq (12 Linien), J = 2 Hz, J = 11.5 Hz, J = 6.5 Hz, 1 H), 4.70 (q, J = 5 Hz, 1 H), 7.19 - 7.26 (m, 1 H), 7.31 - 7.37 ppm (m, 4 H).
   ¹³C-NMR (75 MHz, CDCl₃): δ = 21.1 (q), 21.6 (q), 34.3 (q), 41.0 (t), 68.9 (d), 76.4 (s), 93.6 (d), 125.8 (d), 126.8 (d), 128.6 (d), 144.4 ppm (s).
   MS: m/z (%) = 206 (Spur, M⁺), 191 (10), 147 (16), 146 (51), 145 (53), 131 (61), 121 (14), 118 (24), 117 (21), 105 (100), 103 (11), 91 (21), 77 (30), 51 (11), 45 (18), 43 (60).
(b) Im Siedebereich 101 -106°C fielen 128,5 g Destillat als farblose Flüssigkeit an, die gemäß Gaschromatogramm (60 m DBWax, 50 - 200°C, 4°C/min) 25,8 % 1, 28,9 x 3, 2,7 % 4 und 42,6 % 2 enthielt. Aus dem vorstehenden Material wurde mittels präparativer Gaschromatografie eine analytische Probe von 3/4 im Verhältnis 11:1 isoliert.
   Es ergab sich das folgende - hinsichtlich des Geruchs überraschende - Analyseergebnis für (2RS,4SR,6SR)-2,4,6-Trimethyl-4-phenyl-1,3-dioxan (3):
   Geruch: stark, krautig-frisch, grün, typisch Grapefruit (bestimmt per GC-Sniffing-Analyse). [Anm.: Die neue Substanz (2RS,4SR,6SR)-2,4,6-Trimethyl-4-phenyl-1,3-dioxan (3) ist also ein hervorragender Riechstoff.]
   ¹H-NMR (300 MHz, CDCl₃): δ = 1.15 (d, J = 6.5 Hz, 3 H), 1.37 (d, J = 5 Hz, 3 H), 1.48 (s, 3 H), 2.16 (dd (B-Teil eines AB-Spektrums), J = 8 Hz, J = 14 Hz, 1 H), 2.19 (dd (A-Teil eines AB-Spektrums), J = 5 Hz, J =14 Hz, 1 H), 4.26 (ddq (14 Linien), J = 5 Hz, J = 8 Hz, J = 14 Hz, 1 H), 5.01 (q, J = 5 Hz, 1 H), 7.22 - 7.29 (m, 1 H), 7.33 - 7.38 ppm (m, 4 H).
   MS: m/z (%) = 191 (19, M⁺ - 15), 147 (13), 146 (29), 145 (46), 131 (29), 118 (17), 117 (17), 105 (100), 91 (18), 77 (21), 45 (15), 43 (38).

   Außerdem ergab sich das folgende Analyseergebnis für (2SR,4SR,6RS)-2,4,6-Trimethyl-4-phenyl-1,3-dioxan (4):
   Geruch: nicht wahrnehmbar (bestimmt per GC-Sniffing-Analyse).
   ¹H-NMR (300 MHz, CDCl₃; soweit im Gemisch mit 3 erkennbar): δ = 1,40 (d, J = 5 Hz, 3 H), 1.56 (s, 3 H), 3.72 - 3.85 (m, 1 H), 5.41 ppm (q, J = 5 Hz, 1 H).
   MS: m/z (%) = 191 (19, M⁺ - 15), 162 (36), 147 (17), 145 (51), 121 (17), 118 (11), 117 (14), 106 (11), 105 (100), 103 (11), 91 (16), 77 (26), 51 (10), 45 (14), 43 (36).
(c) Im Siedebereich 106 -108°C fielen 1105 g 2 als farblose Flüssigkeit an; GC-Reinheit: 99,9 %. Bei Abbruch der Destillation verblieben 324 g Sumpfprodukt als schwach gelbliche Flüssigkeit, die gemäß Gaschromatogramm 98,43 % 2, 1,41 % 5 und 0,07 % 6 enthielt.

Es ergab sich das folgende Analyseergebnis für (2RS,4RS,6RS)-2,4,6-Trimethyl-4-phenyl-1,3-dioxan (2):
Geruch: sehr schwach, chemisch-lösemittelartig
¹H-NMR (300 MHz, CDCl₃): δ = 1.22 (d, J = 6 Hz, 3 H), 1.41 (d, J = 5 Hz, 3 H), 1.58 (s, 3 H), 1.60 (dd, J = 11.5 Hz, J = 13 Hz, 1 H), 1.79 (dd, J = 2.5 Hz, J = 13 Hz, 1 H), 4.00 (ddq (12 Linien), J = 2.5 Hz, J = 11.5 Hz, J = 6 Hz, 1 H), 5.18 (q, J = 5 Hz, 1 H), 7.18 - 7.25 (m, 1 H), 7.29 - 7.37 (m, 2 H), 7.42 - 7.46 ppm (m, 2 H).
¹³C-NMR (75 MHz, CDCl₃): δ = 21.6 (q), 21.8 (q), 23.2 (q), 43.6 (t), 68.5 (d), 74.5 (s), 92.2 (d),123.9 (d), 126.6 (d), 128.1 (d), 148.9 ppm (s).
MS: m/z (%) = 206 (0.2, M⁺), 191 (42), 162 (24), 147 (14), 145 (26), 121 (19), 118 (11), 117 (12), 106 (10), 105 (100), 91 (11), 77 (24), 45 (15), 43 (35).

### Beispiel 2:

### Darstellung eines Gemisches der Substanzen 1 und 2 unter Verwendung eines stark sauren Ionenaustauscherharzes:

Zu einer gut gerührten Suspension von 5 g Amberlyst® 15 (trocken, Fa. Aldrich) in 100 g Toluol und 1,5 g Wasser wurde bei 20°C unter gelegentlicher Eiswasserkühlung in 1 h eine Mischung aus 118 g (1 mol) α-Methylstyrol (A) und 88 g (2 mol) Acetaldehyd getropft. Das Reaktionsgemisch wurde über Nacht bei Raumtemperatur nachgerührt. Nach Abfiltration des Katalysators wurde das Lösungsmittel im 20 mbar-Vakuum abdestilliert. Die Destillation des Rückstandes (189,5 g) an einer 30 cm-Metallfüllkörperkolonne im 9 hPa-Vakuum ergab 153,7 g Substanzgemisch 1/2 (74,6 % d.Th.) im Siedebereich 100 - 108°C als farblose Flüssigkeit, die gemäß Gaschromatogramm (30 m DBWAX, 100 - 240°C, 6°C/min) folgende Zusammensetzung aufwies:
54,2 % 1, 0,4 % 3/4, 45,2 % 2, 0,2 % 5.

### Beispiel 3:

### Darstellung eines Gemisches der Substanzen 1 und 2 mit einem hohen Anteil an der Substanz 1 aus α-Methylstyrol (A) und Paraldehyd (B) unter Einwirkung von wässeriger Schwefelsäure:

Zu einem gut gerührten Zweiphasengemisch aus 183 g Hexan-Fraktion (Siedebereich 63-80°C) und 250 g einer Säuremischung aus 210 g Ameisensäure, 12,5 g Schwefelsäure (96 Gew.-%) und 27,5 g Wasser wurde bei 20 - 25°C unter gelegentlicher Eiswasserkühlung in 1 h eine Mischung aus 223 g (1,89 mol) α-Methylstyrol (A) und 183 g (1,385 mol) Paraldehyd (B) getropft. Das Reaktionsgemisch wurde insgesamt 30 h bei 25°C nachgerührt. Die Säurephase wurde abgetrennt, und die organische Phase wurde zweimal mit 100 g Wasser und einmal mit 125 g 5 Gew.-%iger Sodalösung gewaschen. Nach Abdestillation des Lösungsmittels bei Normaldruck verblieben 395 g Rohprodukt, das gemäß Gaschromatogramm (30 m DBWAX, 100 - 240°C, 6°C/min) folgende Zusammensetzung aufwies:
82,0 % 1, 0,5 % 3/4, 15,8 % 2, 1,6 % 5, 0,1 % 6

(Zum Vergleich: Eine analytische Probe, die nach 20 h Nachrührzeit entnommen wurde und wie oben aufgearbeitet wurde, wies folgende gaschromatografische Verteilung auf: 80,5 % 1, 0,5 % 3/4, 17,7 % 2, 1,3 % 5).

Die Destillation des Rohproduktes an einer 40 cm-Metallfüllkörperkolonne erbrachte 341,9 g eines Gemischs der Substanzen 1 und 2 (87,8 % d.Th.) im Siedebereich 100 - 108°C / 9 hPa als farblose Flüssigkeit, die gemäß Gaschromatogramm
83,4 % 1, 0,5 % 3/4 und 16,1 % 2
enthielt. Dieses Produktgemisch erwies sich im Vergleich mit den handelsüblichen Gemischen Vertacetal und Floropal/Corps 717 (vgl. die obige Vergleichsanalyse) als geruchsintensiver und wohlriechender.

Weiterhin fielen 10,3 g Nachlauf im Siedebereich 108 - 111°C / 9 hPa als gelbliche Flüssigkeit an, die gemäß Gaschromatogramm 1,3 % 1, 1,8 % 3/4, 46,7 % 2, 46,1 % 5 und 2,7 % 6 enthielt. Aus dem vorstehenden Material wurden mittels präparativer Gaschromatografie analytische Mengen der reinen Dihydropyrane 5 und 6 isoliert.

Es ergab sich das folgende Analyseergebnis für 3,6-Dthydro-cis-2,6-dimethyl-4-phenyl-2H-pyran (5):
Geruch: nicht wahrnehmbar (bestimmt per GC-Sniffing-Analyse).
¹H-NMR (300 MHz, CDCl₃): δ = 1.33 (d, J = 7 Hz, 3 H), 1.36 (d, J = 6 Hz, 3H), 2.32 - 2.36 (m, 2 H), 3.81 (sextett, J = Hz, 1 H), 4.40 - (m, 1 H), 6.01 (q, J = 1.5 Hz, 1H), 7.23 - 7.43 ppm (m, 5 H).
¹³C-NMR (75 MHz, CDCl₃): δ = 21.6 (q), 21.7 (q), 34.6 (t), 70.2 (d), 71.3 (d), 124.8 (d), 127.2 (2 d), 128.4 (d), 134.2 (s), 140.2 ppm (s).
MS: m/z (%) = 188 (69, M⁺), 173 (39), 159 (53), 145 (91), 131 (78), 130 (41), 129 (54), 128 (41), 117 (27), 115 (30), 103 (26), 91 (35), 43 (100).

Und schließlich ergab sich das folgende Analyseergebnis für 3,6-Dihydro-trans-2,6-dimethyl-4-phenyl-2H-pyran (6):
Geruch: sehr schwach, chemisch-technisch (bestimmt per GC-Sniffing-Analyse).
¹H-NMR (300 MHz, CDCl₃): δ = 1.32 (d, J = 6 Hz, 3 H), 1.33 (d, J = 7 Hz, 3 H), 2.25 (ddt, J = 2.5 Hz, J = 8.5 Hz, J = 16.5 Hz, 1 H), 2.43 (ddt, J = 1 Hz, J = 3.5 Hz, J = 16.5 Hz, 1H), 4.00 (m, 1 H), 4.55 (m, 1 H), 6.06 (m, 1 H), 7.22 - 7.40 ppm (m, 5 H).
MS: m/z (%) = 188 (66, M⁺), 173 (49), 159 (55), 145 (90), 131 (77), 130 (39), 129 (56), 128 (43), 115 (29), 103 (32), 91 (35), 43 (100).

### Beispiel 4:

### Herstellung von Gemischen aus (2RS,4SR,6RS)-2,4,6-Trimethyl-4-phenyl-1,3-dioxan (1) und (2RS,4RS,6RS)-2,4,6-Trimethyl-4-phenyl-1,3-dioxan (2) mit einem erhöhten Anteil des Isomeren 1:

### Alternative a:

### Herstellung aus einem Gemisch der Substanzen 1 und 2 mit einem Isomerenverhältnis 1/2 = 1,2 unter Einwirkung von Bortrifluorid-Ethyletherat

25 g des Gemischs 1/2 gemäß Beispiel 2 wurden in 25 g abs. CH₂Cl₂ gelöst und bei 20°C in einer Stickstoffatmosphäre unter Rühren mit 0,5 g Bortrifluorid-ethyletherat versetzt. Die sich tief dunkelgelb verfärbende Reaktionsmischung wurde 7 h bei Raumtemperatur nachgerührt. Dann wurde die Reaktionsmischung mit 5 Gew.-%iger Sodalösung neutralgewaschen und bei Normaldruck vom Lösungsmittel befreit. Nach Kugelrohrdestillation des verbliebenen Rückstandes (24,3 g) im 3 hPa-Vakuum erhielt man 23,4 g Produkt als farblose Flüssigkeit, die gemäß Gaschromatogramm
72,5 % 1, 4,5 % 4-Phenylbutan-2-on, 0,5 % 3/4, 12,6 % 2, 8,5 % 5 und 1,2 % 6
enthielt.

### Alternative b:

### Herstellung aus (2RS,4RS,6RS)-2,4,6-Trimethyl-4-phenyl-1,3-dioxan (2) unter Einwirkung von wässeriger Schwefelsäure

Eine Lösung von 250 g 2 (aus Beispiel 1; GC-Reinheit: 99,9 %) in 250 g Hexan-Fraktion (Siedebereich 63-80°C) wurde unter intensivem Rühren mit 250 g einer Säuremischung aus 220 g Ameisensäure, 2,5 g Schwefelsäure (96 Gew.-%) und 27,5 g Wasser in 10 min bei 20°C versetzt und über Nacht (15 h) bei Raumtemperatur nachgerührt. Das Zweiphasensystem wurde analog Beispiel 3 aufgearbeitet. Es verblieben 248 g Rohprodukt, das gemäß Gaschromatogramm (30 m DBWAX, 100 - 240°C, 6°C/min) folgende Zusammensetzung aufwies:
80,5 % 1, 0,6 % 3/4, 15,0 % 2, 3,7 % 5, 0,2 % 6.

Die Destillation des Rohproduktes an einer 40 cm-Metallfüllkörperkolonne erbrachte 233,3 g 1/2 (93,3 % d.Th.) im Siedebereich 100 - 108°C / 9 hPa als farblose Flüssigkeit, die gemäß Gaschromatogramm
83,8 % 1, 0,6 % 3/4 und 15,6 % 2
enthielt.

### Beispiel 5:

### Testreihe: Variationen eines Parfümöls vom Citrus-Typ / sensorische Auswirkungen der Anwesenheit der Substanzen 1 und/oder 2 in verschiedenen Konzentrationen und Konzentrationsverhältnissen

Zusatz einer festen Menge 1 + 2 mit variablen Anteilen von 1 und 2

**Tabelle 2**

| | Parfümöl-Mischungen | | | | | |
|---|---|---|---|---|---|---|
| Parfümöl -Komponenten | a | b | c | d | e | f |
| Thiocineol 1 %ig | 10 | 10 | 10 | 10 | 10 | 10 |
| Oxane* 10 %ig | 2 | 2 | 2 | 2 | 2 | 2 |
| cis-3-Hexenol 10 %ig | 5 | 5 | 5 | 5 | 5 | 5 |
| Hivertal 10 %ig | 10 | 10 | 10 | 10 | 10 | 10 |
| Aldehyd C10 | 10 | 10 | 10 | 10 | 10 | 10 |
| Aldehyd C12 | 5 | 5 | 5 | 5 | 5 | 5 |
| Dihydromyrcenol | 50 | 50 | 50 | 50 | 50 | 50 |
| Geranylnitril | 10 | 10 | 10 | 10 | 10 | 10 |
| Orangenöl brasilianisch | 200 | 200 | 200 | 200 | 200 | 200 |
| Citronenöl italienisch pelatrice | 360 | 360 | 360 | 360 | 360 | 360 |
| Citral | 100 | 100 | 100 | 100 | 100 | 100 |
| Terpinolen 20 | 108 | 108 | 108 | 108 | 108 | 108 |
| Litsea Cubebaöl dest. | 100 | 100 | 100 | 100 | 100 | 100 |
| Allylcapronat | 10 | 10 | 10 | 10 | 10 | 10 |
| Isomer **2** (pur) | - | 30 | - | - | - | - |
| Mischung **1/2** (55:45) | - | - | 30 | - | - | - |
| Mischung **1/2** (65:35) | - | - | - | 30 | - | - |
| Mischung **1/2** (80:20) | - | - | - | - | 30 | - |
| Isomer **1** (pur) | - | - | - | - | - | 30 |

| | | | | | | |
|---|---|---|---|---|---|---|
| *Produkt der Fa. Firmenich (Handelsname) | | | | | | |

Die Mischung a stellt eine Citrus-Grundmischung dar. Sie enthält weder
(2RS,4SR,6RS)-2,4,6-Trimethyl-4-phenyl-1,3-dioxan (1) noch
(2RS,4RS,6RS)-2,4,6-Trimethyl-4-phenyl-1,3-dioxan (2).

Mischung b enthält im Vergleich mit Mischung a zusätzlich 3 % der Substanz 2. Die Mischung b riecht geringfügig weniger intensiv als die Grundmischung a.

Bei alternativer Anwesenheit von 3 % einer 55:45-Mischung 1/2 (Mischung c) bzw. von 3 % einer 65:35-Mischung 1/2 (Mischung d) gewinnt das Parfümöl mit steigender Konzentration von 1 an Frische und Natürlichkeit gegenüber den flacher wirkenden Mischungen a und b.

Bei alternativer Anwesenheit von 3 % einer 80:20-Mischung 1/2 (Mischung e) bzw. 3 % der reinen Substanz 1 (Mischung f) tritt dieser positive Effekt in einer Weise verstärkt auf, die die Auswirkung der bloßen Konzentrationssteigerung an 1 nachhaltig übertrifft und als überproportional gesteigerte natürliche Ausstrahlung der Mischungen e und f gegenüber den Mischungen c und d wahrzunehmen ist, die merklich weniger grün und schalig riechen.

### Beispiel 6:

### Testreihe: Variationen eines Parfümöls vom Citrus-Typ / sensorische Auswirkung der Anwesenheit der Substanz 2 in verschiedenen Konzentrationen neben gleichbleibenden Konzentrationen der Substanz 1

Zusatz einer festen Menge 1 und variabler Mengen an 2 gemäß der nachfolgenden

**Tabelle 3**

| | Parfümöl-Mischungen | | | | |
|---|---|---|---|---|---|
| Parfümöl-Komponenten | a | g | h | i | f |
| Thiocineol 1%ig | 10 | 10 | 10 | 10 | 10 |
| Oxane* 10%ig | 2 | 2 | 2 | 2 | 2 |
| cis-3-Hexenol 10 %ig | 5 | 5 | 5 | 5 | 5 |
| Hivertal 10 %ig | 10 | 10 | 10 | 10 | 10 |
| Aldehyd C10 | 10 | 10 | 10 | 10 | 10 |
| Aldehyd C12 | 5 | 5 | 5 | 5 | 5 |
| Dihydromyrcenol | 50 | 50 | 50 | 50 | 50 |
| Geranylnitril | 10 | 10 | 10 | 10 | 10 |
| Orangenöl brasilianisch | 200 | 200 | 200 | 200 | 200 |
| Citronenöl italienisch pelatrice | 350 | 350 | 350 | 350 | 350 |
| Citral | 100 | 100 | 100 | 100 | 100 |
| Terpinolen 20 | 108 | 108 | 108 | 108 | 108 |
| Litsea Cubebaöl dest. | 100 | 100 | 100 | 100 | 100 |
| Allylcapronat | 10 | 10 | 10 | 10 | 10 |
| Mischung **1/2** (50:50) | - | 60 | - | - | - |
| Mischung **1/2** (60:40) | - | - | 50 | - | - |
| Mischung **1/2** (80:20) | - | - | - | 37,5 | - |
| Isomer **1** (pur) | - | - | - | - | 30 |

| | | | | | |
|---|---|---|---|---|---|
| *Produkt der Fa. Firmenich (Handelsname) | | | | | |

Die Mischung a ist die Citrus-Grundmischung, die auch in der Testreihe gemäß Beispiel 5 Verwendung fand; die Mischung f wurde ebenfalls bereits in dieser Testreihe eingesetzt.

Die Mischungen g, h, i und f enthalten jeweils 30 Mengenanteile der Substanz 1 und unterschiedliche Mengenanteile der Substanz 2. Jede der Mischungen g, h, i und f riecht im Vergleich mit der Grundmischung a frischer und natürlicher als diese.

Im Vergleich der Mischungen g, h, i und f untereinander ist mit fallender Konzentration der Substanz 2 (von g über h und i nach f) eine signifikante, vorteilhafte Verstärkung des natürlichen grünen Schalengeruchs feststellbar, wobei gleichzeitig in der Reihenfolge g, h, i und f die Ausstrahlung und Ausgewogenheit des Parfümöls in gewünschter Weise zunimmt.

### Beispiel 7:

### Parfümöl (Damennote) gemäß nachfolgender

**Tabelle 4:**

| | a | b |
|---|---|---|
| Helional | 15 | 15 |
| Heliotropin | 5 | 5 |
| Hydroxycitronellal | 40 | 40 |
| Methyliridon gamma | 200 | 200 |
| Phenylethylalkohol | 100 | 100 |
| Base Jasmin 231* | 15 | 15 |
| Base Rose de Mai* | 15 | 15 |
| Hedione^{c}** | 40 | 40 |
| Lilial^{c}*** | 15 | 15 |
| Lyral^{c}**** | 60 | 60 |
| Patchouliöl indonesisch rektifiziert | 60 | 60 |
| Habanolide^{c}** | 60 | 60 |
| Exaltolide^{c}** | 30 | 30 |
| Linalool | 20 | 20 |
| Undecalacton-gamma | 20 | 20 |
| Eugenol | 10 | 10 |
| Geraniol | 10 | 10 |
| Hexylacetat | 10 | 10 |
| Methyloctincarbonat 1 %ig | 10 | 10 |
| Vanillin | 10 | 10 |
| Allylcyclohexanpropionat 10 %ig | 5 | 5 |
| Anethol | 5 | 5 |
| Cassis Bourgeons absolue 1 %ig | 5 | 5 |
| Cedernblätteröl 10 %ig | 5 | 5 |
| cis-3-Hexenylacetat 10 %ig | 5 | 5 |
| Citronellol | 5 | 5 |
| Cumarin | 5 | 5 |
| Dimethylbenzylcarbinylacetat | 5 | 5 |
| Ethylcarprylat 10 %ig | 5 | 5 |
| Isoeugenylacetat | 5 | 5 |
| Phenyldimethylcarbinol | 5 | 5 |
| Styraxöl 10 %ig | 5 | 5 |
| Violet leaf absolue 10 %ig | 5 | 5 |
| Iris absolue 10 %ig | 2 | 2 |
| Weinhefenöl grün 10 %ig | 5 | 5 |
| Ylang-Öl | 5 | 5 |
| Cassis 345** 10 %ig | 3 | 3 |
| Rosenoxid-L* 10 %ig | 3 | 3 |
| Damascon-alpha** | 2 | 2 |
| Decalacton-gamma | 2 | 2 |
| Ethylvanillin | 2 | 2 |
| Evernyl^{c}*** | 2 | 2 |
| Ethylcapronat 10 %ig | 3 | 3 |
| Isoamylacetat 10 %ig | 2 | 2 |
| Jasmin absolue | 5 | 5 |
| Rose absolue Marocco | 2 | 2 |
| Rosenöl bulgarisch | 2 | 2 |
| Dipropylenglykol | 150 | 130 |
| Isomere 1/2 (gemäß Beispiel 3) | - | 20 |

| | | |
|---|---|---|
| * Dragoco-Produkt (Handelsname) | | |
| ** Firmenich-Produkt (Handelsname) | | |
| *** Givaudan-Produkt (Handelsname) | | |
| **** IFF-Produkt (Handelsname) | | |

Die Mischung a stellt eine Parfümölvariation vom Typ Yvresse (vormals "Champagne" genannt) dar. Sie besitzt eine fruchtige, anisige Kopfnote mit Grünelementen, einen blumigen Mittelteil mit Nelken-Aspekten und einen pudrig-holzigen Fond mit Moschus- und Eichenmoos-Charakter. Durch Austausch von 2 % Dipropylenglykol in Mischung a gegen 2 % des Substanzgemischs 1/2 gemäß Beispiel 3 wird Mischung b erhalten. Im Vergleich zeichnet sich Mischung b gegenüber a im Angeruch durch eine spritzig-leichte, fruchtige Agrumennote aus. Der Geruchsablauf von b wirkt insgesamt feiner ausgewogen, und im Nachgeruch erweist sich b gegenüber a als strahlender und substantiver.

### Beispiel 8:

### Parfümöl (Herrennote) für Eau sauvage gemäß nachfolgender

**Tabelle 5:**

| | a | b |
|---|---|---|
| Hexylzimtaldehyd, alpha | 20 | 20 |
| Helional | 15 | 15 |
| Aldehyd C8 | 5 | 5 |
| Aldehyd C10 | 5 | 5 |
| Aldehyd C12 | 3 | 3 |
| Bergamottöl spezial* | 120 | 120 |
| Bergamottöl Reggio | 60 | 60 |
| Bergamottöl italienisch | 40 | 40 |
| Analine** 10 %ig | 2 | 2 |
| Linalool | 70 | 70 |
| Linalylacetat | 60 | 60 |
| Evernyl ^{c}*** | 30 | 30 |
| Eichenmoosextrakt grün jugoslawisch | 10 | 10 |
| Citral | 40 | 40 |
| Citronenöl italienisch pelatrice | 30 | 30 |
| Lavandinöl Abrialis | 30 | 30 |
| Lavendelöl französisch | 20 | 20 |
| Orangenöl Guinea | 15 | 15 |
| Limetteöl destilliert | 10 | 10 |
| Hedione ^{c}**** | 20 | 20 |
| Methyliridon gamma | 20 | 20 |
| Patchouliöl indonesisch rektifiziert | 20 | 20 |
| Rosenholzöl brasilianisch | 20 | 20 |
| [2'S-(2'α,4'aα,8'aα)]-Hexahydro-1',1',5',5'-tetramethylspiro[1,3-dioxolan-2,8'(5'H)-[2H-2,4a]methanonaphthalin]* | 15 | 15 |
| Base Amber* | 15 | 15 |
| Basilikumöl Comoren | 15 | 15 |
| Corianderöl | 15 | 15 |
| Nelkenöl rektifiziert | 10 | 10 |
| Rosmarinöl tunesisch | 10 | 10 |
| Cumarin | 15 | 15 |
| Eugenol | 15 | 15 |
| Base Jasmin 231* | 10 | 10 |
| Benzylacetat | 10 | 10 |
| Diheptylacetat* | 10 | 10 |
| Litsea Cubebaöl destilliert | 10 | 10 |
| Methylchavicol | 10 | 10 |
| Myrtenöl | 10 | 10 |
| Sandranol^{c}* | 10 | 10 |
| Amylvinylcarbinylacetat 10 %ig | 5 | 5 |
| Angelikawurzelöl 10 %ig | 5 | 5 |
| Carvon-L | 5 | 5 |
| Cuminöl | 5 | 5 |
| Geraniol | 5 | 5 |
| Geraniumöl Bourbon | 5 | 5 |
| Geranylacetat | 5 | 5 |
| Geranylnitril 10 %ig | 5 | 5 |
| Habanolide^{c}**** | 5 | 5 |
| Iris absolue 10 %ig | 5 | 5 |
| Nerylacetat | 5 | 5 |
| Opoponax-Extrakt | 5 | 5 |
| Opoponaxöl 10%ig | 2 | 2 |
| Olibanumöl | 2 | 2 |
| Sandelholzöl ostindisch | 2 | 2 |
| Petitgrainöl Paraguay | 5 | 5 |
| Rosenoxid inaktiv high cis* 10 %ig | 5 | 5 |
| Terpineol | 5 | 5 |
| Vanillin 10 %ig | 5 | 5 |
| Vetiveröl | 5 | 5 |
| Vetiverylacetat | 5 | 5 |
| Zimtblätteröl | 5 | 5 |
| Citronellol | 3 | 3 |
| Heliotropin | 3 | 3 |
| Krauseminzöl amerikanisch rektifiziert | 3 | 3 |
| Ocimen | 3 | 3 |
| Undecalacton-gamma | 3 | 3 |
| Allycyclohexanpropionat 10 %ig | 2 | 2 |
| Bayöl 10 %ig | 2 | 2 |
| Hexylmethylether | 2 | 2 |
| Indol | 2 | 2 |
| Menthol-L | 2 | 2 |
| Novorosan^{c}* 10 %ig | 2 | 2 |
| Oct-1-en-3-ol 10 %ig | 2 | 2 |
| Dipropylenglykol | 25 | 5 |
| Isomere 1/2 (gemäß Beispiel 3) | - | 20 |

| | | |
|---|---|---|
| * Dragoco-Produkt (Handelsname) | | |
| ** H&R Florasynth-Produkt (Handelsname) | | |
| *** Givaudan-Produkt (Handelsname) | | |
| **** Firmenich-Produkt (Handelsname) | | |

Die Mischung a stellt in 10 %iger alkoholischer Lösung eine klassische Eau-sauvage-Variation dar. Sie besitzt eine agrumige Kopfnote, einen blumigen Mittelteil mit würzigen Beinoten und einen holzigen Fond mit moosigen und moschusartigen Anklängen. Durch Austausch von 2% Dipropylenglykol in Mischung a gegen 2 % des Substanzgemischs 1/2 gemäß Beispiel 3 wird Mischung b erhalten. Im Vergleich der 10 %igen alkoholischen Lösungen zeichnet sich Mischung b gegenüber a durch eine erheblich frischere, positiv angehobene Kopfnote gepaart mit einer feinen Gardenien-Note im Mittelteil aus. Der Geruchsablauf von b wirkt insgesamt heller, klarer und ausgewogener, und im Nachgeruch von b tritt gegenüber a eine sehr erwünschte, natürliche, weiche Ausstrahlung in den Vordergrund.

## Patentansprüche

1. Verbindung der allgemeinen Formel
(2RS,4SR,6X)-2,4,6-Trimethyl-4-phenyl-1,3-dioxan
mit dem Konfigurationsmerkmal X = SR (Verbindung 3):

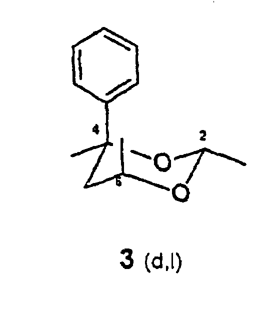

2. Verfahren zur Herstellung von (2RS,4SR,6RS)-2,4,6-Trimethyl-4-phenyl-1,3-dioxan (1),
**dadurch gekennzeichnet, daß** ein Gemisch der Verbindungen
(2RS,4SR,6RS)-2,4,6-Trimethyl-4-phenyl-1,3-dioxan 1 und
(2RS,4RS,6RS)-2,4,6-Trimethyl-4-phenyl-1,3-dioxan 2 mit Säure behandelt wird, vorzugsweise mit einer Brönstedt-Säure bei 20 - 30°C.

3. Verfahren zur Herstellung von (2RS,4SR,6RS)-2,4,6-Trimethyl-4-phenyl-1,3-dioxan (1),
**dadurch gekennzeichnet, daß** (2RS,4RS,6RS)-2,4,6-Trimethyl-4-phenyl-1,3-dioxan (2) als Ausgangsmaterial eingesetzt und vorzugsweise mit einer Brönstedt-Säure bei 20 - 30°C behandelt wird.

4. Verwendung der Verbindung der allgemeinen Formel
(2RS,4SR,6X)-2,4,6-Trimethyl-4-phenyl-1,3-dioxan
mit dem Konfigurationsmerkmal X = RS (Verbindung 1) oder SR (Verbindung 3) als Riechstoff bzw. Bestandteil von Riechstoffmischungen für kosmetische und technische Anwendungen:

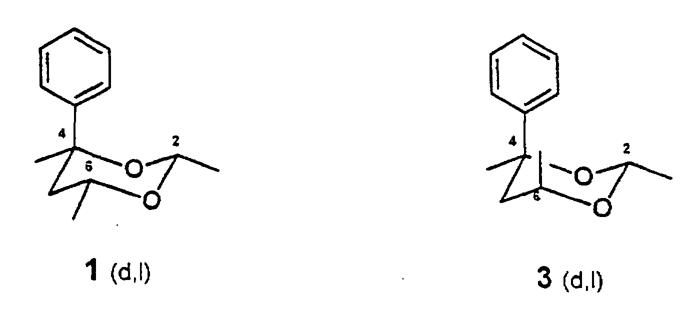

5. Riechstoffgemisch, umfassend
(2RS,4SR,6RS)-2,4,6-Trimethyl-4-phenyl-1,3-dioxan (1) und
(2RS,4RS,6RS)-2,4,6-Trimethyl-4-phenyl-1,3-dioxan (2),
**dadurch gekennzeichnet, daß**
(2RS,4SR,6RS)-2,4,6-Trimethyl-4-phenyl-1,3-dioxan (1) und
(2RS,4RS,6RS)-2,4,6-Trimethyl-4-phenyl-1,3-dioxan (2)
in einem Verhältnis von mindestens 4:1 vorliegen.

6. Riechstoffgemisch, umfassend
(2RS,4SR,6RS)-2,4,6-Trimethyl-4-phenyl-1,3-dioxan (1),
**dadurch gekennzeichnet, daß** das Riechstoffgemisch zumindest 80 Gewichtsprozent (2RS,4SR,6RS)-2,4,6-Trimethyl-4-phenyl-1,3-dioxan (1) umfaßt.

7. Verwendung eines Gemischs nach einem der Ansprüche 6 oder 7 als Riechstoff bzw. Bestandteil von Riechstoffmischungen für kosmetische und technische Anwendungen.

## Claims

1. Compound of the general formula
(2RS,4SR,6X)-2,4,6-trimethyl-4-phenyl-1,3-dioxane
with the configurational feature X = SR (compound 3):

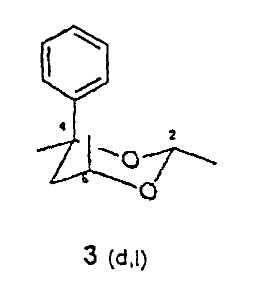

2. Process for the production of (2RS,4SR,6RS)-2,4,6-trimethyl-4-phenyl-1,3-dioxane (1),
**characterised in that** a mixture of the compounds
(2RS,4SR,6RS)-2,4,6-trimethyl-4-phenyl-1,3-dioxane 1 and
(2RS,4RS,6RS)-2,4,6-trimethyl-4-phenyl-1,3-dioxane 2 is treated with acid, preferably with a Brönsted acid at 20 - 30°C.

3. Process for the production of (2RS,4SR,6RS)-2,4,6-trimethyl-4-phenyl-1,3-dioxane (1),
**characterised in that** (2RS,4RS,6RS)-2,4,6-trimethyl-4-phenyl-1,3-dioxane (2) is used as the starting material and is preferably treated with a Brönsted acid at 20 - 30°C.

4. Use of the compound of the general formula
(2RS,4SR,6X)-2,4,6-trimethyl-4-phenyl-1,3-dioxane
with the configurational feature X = RS (compound 1) or SR (compound 3) as a fragrance or a component of fragrance mixtures for cosmetic and industrial applications:

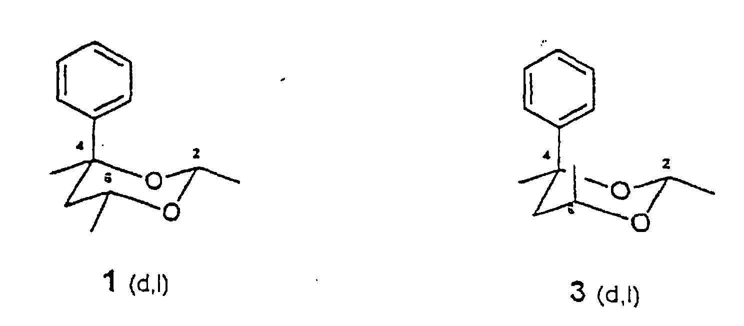

5. Fragrance mixture comprising
(2RS,4SR,6RS)-2,4,6-trimethyl-4-phenyl-1,3-dioxane (1) and
(2RS,4RS,6RS)-2,4,6-trimethyl-4-phenyl-1,3-dioxane (2),
**characterised in that**
(2RS,4SR,6RS)-2,4,6-trimethyl-4-phenyl-1,3-dioxane (1) and
(2RS,4RS,6RS)-2,4,6-trimethyl-4-phenyl-1,3-dioxane (2) are present in a ratio of at least 4:1.

6. Fragrance mixture comprising
(2RS,4SR,6RS)-2,4,6-trimethyl-4-phenyl-1,3-dioxane (1),
**characterised in that** the fragrance mixture comprises at least 80 wt.% (2RS,4SR,6RS)-2,4,6-trimethyl-4-phenyl-1,3-dioxane (1).

7. Use of a mixture according to one of claims 6 or 7 as a fragrance or a component of fragrance mixtures for cosmetic and industrial applications.

## Revendications

1. Composé de formule générale (2RS, 4SR, 6X)-2,4,6-triméthyl-4-phényle-1,3-dioxane présentant la caractéristique de configuration X = SR (composé 3) :

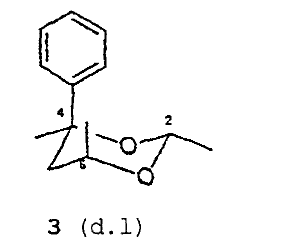

2. Procédé de préparation du (2RS, 4SR, 6RS)-2,4,6-triméthyl-4-phényl-1,3-dioxane (1), **caractérisé en ce qu'**un mélange des composés (2RS, 4SR, 6RS)-2,4,6-triméthyl-4-phényl-1,3-dioxane 1 et (2RS, 4RS, 6RS)-2,4,6-triméthyl-4-phényl-1,3-dioxane 2 est traité à l'acide, de préférence avec un acide de Brönstedt à une température allant de 20 à 30 °C.

3. Procédé de préparation du (2RS, 4SR, 6RS)-2,4,6-triméthyl-4-phényle-1,3-dioxane (1), **caractérisé en ce que** le (2RS, 4RS, 6RS)-2,4,6-triméthyl-4-phényl-1,3-dioxane (2) est utilisé comme produit de départ et traité de préférence avec un acide de Brönstedt, à une température allant de 20 à 30 °C.

4. Utilisation du composé de formule générale (2RS, 4SR, 6X)-2,4,6-triméthyl-4-phényl-1,3-dioxane, ayant la caractéristique de configuration X = RS (composé 1) ou SR (composé 3), en tant que parfum ou en tant que composant de mélanges de parfums destinés à des applications techniques et cosmétiques :

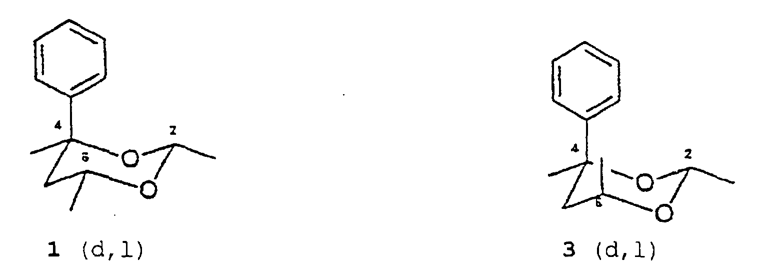

5. Mélange de parfums, comprenant : (2RS, 4SR, 6RS)-2,4,6-triméthyl-4-phényl-1,3-dioxane (1) et (2RS, 4RS, 6RS)-2,4,6-triméthyl-4-phényl-1,3-dioxane (2),
**caractérisé en ce que**
le (2RS, 4SR, 6RS)-2,4,6-triméthyl-4-phényle-1,3-dioxane (1) et le (2RS, 4RS, 6RS)-2,4,6-triméthyl-4-phényle-1,3-dioxane (2) sont présents dans un rapport d'au moins 4/1.

6. Mélange de parfums comprenant du (2RS, 4SR, 6RS)-2,4,6-triméthyl-4-phényl-1,3-dioxane (1), **caractérisé en ce que** le mélange de parfums comprend au moins 80 pour cent en poids de (2RS, 4SR, 6RS)-2,4,6-triméthyl-4-phényle-1,3-dioxane (1).

7. Utilisation d'un mélange selon l'une quelconque des revendications 6 ou 7 en tant que parfum ou en tant que composant de mélanges de parfums destinés à des applications cosmétiques et techniques.
